Europäisches Patentamt

European Patent Office

Office européen des brevets

(11) Veröffentlichungsnummer: **0 008 727**
**A1**

(12) **EUROPÄISCHE PATENTANMELDUNG**

(21) Anmeldenummer: **79103034.9**

(22) Anmeldetag: **20.08.79**

(51) Int. Cl.³: **C 07 B 1/00**
**B 01 J 31/02**
//C07C5/03, C07C5/10,
C07C13/18, C07C29/14,
C07C29/17, C07C29/20,
C07C29/136, C07C31/10,
C07C31/12

(30) Priorität: **24.08.78 DE 2837022**

(43) Veröffentlichungstag der Anmeldung:
**19.03.80 Patentblatt 80/6**

(84) Benannte Vertragsstaaten:
**BE CH DE FR GB IT**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Kümmerle, Kurt, Dr.**
**Unter den Eichen 1**
**D-6233 Kelkheim, (Taunus)(DE)**

(72) Erfinder: **Heise, Hartmut, Dr.**
**Am Rehsteig 8**
**D-6232 Bad Soden am Taunus(DE)**

(54) **Verfahren zur Hydrierung organischer Verbindungen.**

(57) Die bekannte katalytische Hydrierung von
N-Acetoacetylarylamiden zu 3-Hydroxybuttersäure-
arylamiden mit Phosphinen als Promotoren läßt sich auf die
Hydrierung anderer organischer Verbindungen übertragen,
wobei auch Phosphinoxide als Promotoren dienen können.

EP 0 008 727 A1

- 1 -

HOECHST AKTIENGESELLSCHAFT    HOE 78/F 174      Dr.KL/wö

Verfahren zur Hydrierung organischer Verbindungen

Aus der DE-OS 26 09 835 ist ein Verfahren zur Herstellung
von 3-Hydroxybuttersäurearylamiden durch katalytische
Hydrierung von N-Acetoacetylarylamiden in flüssiger Phase
bekannt, bei dem die Hydrierung unter Zusatz von Aminen,
Phosphinen oder Trialkylphosphaten als Promotoren durchgeführt wird. Es wurde nun gefunden, daß organische Phosphine
auch bei anderen Hydrierungen als Promotoren wirken und
weiterhin, daß auch die entsprechenden Phosphinoxide eine
solche Wirkung aufweisen.

Diese Eignung der Phosphine als Promotoren für Hydrierungen
allgemein ist in sofern überraschend, als aus der US-PS
3 642 658 bekannt ist, daß solche Phosphine auf Hydrierungskatalysatoren aus Metallen der 8. Gruppe des Peridensystems dann als Katalysatorgifte wirken, wenn man die
Kontakte mit diesen Phosphinen vorbehandelt. Die so vorbehandelten Katalysatoren zeigen dann eine reduzierte selektive Aktivität.

Ein Vorurteil gegen die Übertragbarkeit der Lehre der
DE-OS 26 09 835 auf andere Hydrierungen war auch der Veröffentlichung von H. Adkins u. H.R. Billica, J.Amer. Chem.Soc. 70 (1948), 695-698, zu entnehmen, wo beschrieben ist, daß der Zusatz von Triethylamin zwar die Hydrierung von Ketoverbindungen fördert, jedoch die Hydrierung benzoider Kerne hemmt.

Die Tatsache, daß Promotoren in sehr unterschiedlicher Weise auf Katalysatoren einwirken, ist vielfach beschrieben worden. Hinsichtlich organischer Promotoren kann auf die US-PS 3 145 231 verwiesen werden, die die katalytische Hydrierung von halogensubstituierten aromatischen Nitroverbindungen zu den entsprechenden halogensubstituierten Aminen mit Platinkatalysatoren und Stickstoffbasen, wie Morpholin, betrifft. Andererseits ist es aus K. Kindler u.a., Ann. 605 (1957), 200, bekannt, daß Morpholin bei der katalytischen Reduktion von $\alpha$-Arylcarbinolen zu den entsprechenden Kohlenwasserstoffen inhibierend wirkt.

Gegenstand der Erfindung ist somit ein Verfahren zur katalytischen Hydrierung organischer Verbindungen in flüssiger Phase unter Verwendung von organischen Phosphorverbindungen als Promotoren, dadurch gekennzeichnet, daß als Promotor ein organisches Phosphin oder Phosphinoxid eingesetzt wird, wobei die Hydrierung von N-Acetoacetylarylamiden mit Phosphinen als Promotoren ausgenommen ist.

Die erfindungsgemäß einzusetzenden Promotoren aktivieren alle katalytischen Hydrierungen, d.h. sowohl Hydrogenisierungen, also die Anlagerung von Wasserstoff an ungesättigte Systeme ohne Molekülspaltung, als auch Hydrogenolysen, also Einlagerungsreaktionen von Wasserstoff unter Spaltung des Moleküls. Diese Verstärkung der katalytischen Wirkung ist nicht auf einzelne heterogene Hydrierungskatalysatoren beschränkt, sondern zeigt sich generell in einer Erhöhung der Reaktionsgeschwindigkeit bzw. in einer Erniedrigung von Temperatur und/oder Druck des Reaktionssystems, so daß damit die Voraussetzungen gegeben sind, eine Hydrierung unter milden und schonenden Bedingungen durchzuführen. Solche Bedingungen werden in solchen Fällen gefordert, wo störende Neben- und/oder Folgereaktionen die Ausbeute des Endproduktes schmälern und gegebenenfalls dessen Qualität beeinträchtigen, wie beispielsweise bei der katalytischen Reduktion halogensubstituierter aromatischer Nitroverbindungen. Die erfindungsgemäß einzusetzenden Promotoren ermöglichen auch eine Erniedrigung der Katalysatorkonzentration und bewirken eine Erhöhung der Lebensdauer des Katalysators, so daß dieser längere Zeit benutzt bzw. häufiger in das Reaktionssystem zurückgeführt werden kann.

Im Folgenden werden einige bevorzugte Ausgestaltungen der Erfindung näher beschrieben:

Als Phosphine bzw. Phosphinoxide kommen alle Verbindungen in Betracht, die drei gleiche oder verschiedene über ein Kohlenstoffatom an das Phosphoratom gebundene Reste enthalten. Als solche Reste kommen Alkylreste mit bis zu etwa 20 Kohlenstoffatomen, Cycloalkylreste, insbesondere Cyclohexyl, Aralkylreste, insbesondere durch Phenyl substituierte niedere Alkylreste, wie Benzyl oder Phenethyl, sowie Arylreste, insbesondere Phenylreste, in Betracht. Bevorzugte Promotoren sind Trialkylphosphinoxide und insbesondere Trialkylphosphine mit mittleren Alkylresten von 4 bis 12 Kohlenstoffatomen sowie Triphenylphosphin.

Die Promotoren werden dem Ausgangsmaterial bzw. der Lösung des Ausgangsmaterials in einer Konzentration von bis zu 10 %, vorzugsweise 0,1 bis 5 %, insbesondere 0,25 bis 2,5 %, bezogen auf die Masse des Ausgangsmaterials, eingesetzt.

Sofern die Hydrierung in Gegenwart eines Lösemittels durchgeführt wird, wird hierfür zweckmäßig ein Lösemittel eingesetzt, das das Ausgangsmaterial und das Endprodukt zu lösen vermag. Bevorzugt sind wassermischbare Lösemittel.

- 4 -                                    0008727

Als Hydrierungskatalysatoren kommen bspw. übliche Trägerkatalysatoren        aus Nickel, Kobalt, Palladium, Platin, Rhodium, Iridium und Ruthenium bzw. übliche Skelett-
Katalysatoren, bspw. aus Nickel oder Kobalt, in Betracht.

Die Hydrierung wird bei Temperaturen bis zu 250°C,
vorzugsweise jedoch bei niedrigeren Temperaturen von bis
zu etwa 150°C durchgeführt. Der Druck wird in Abhängigkeit vom zu hydrierenden Material und der Temperatur
gewählt und kann bis zu 200 bar betragen, vorzugsweise
jedoch bis zu etwa 60 bar. Der Einsatz der erfindungsgemäßen Promotoren bringt hier den Vorteil mit sich, daß
relativ niedrige Drücke angewandt werden können und somit
teuere Hochdruckapparaturen unnötig sind.

Die Hydrierungen werden in bekannter Weise durchgeführt.
Die Aufarbeitung richtet sich nach den jeweiligen Gegebenheiten: Die Abtrennung des Katalysators aus der Reaktionsmischung erfolgt durch Filtrieren oder Dekantieren, wobei
anhaftende Reste des Reaktionsgemisches zweckmäßig durch
Auswaschen abgetrennt werden. Aus dem Filtrat wird das
Endprodukt in üblicher Weise isoliert, beispielsweise -
gegebenenfalls nach Abdestillieren des Lösemittels - als
Feststoff isoliert bzw. abdestilliert. Wenn das Hydrierungsprodukt in Wasser wenig löslich ist und ein wassermischbares Lösemittel eingesetzt wurde, kann das Produkt
auch aus dem Filtrat durch Verdünnen mit Wasser abgeschieden werden, mit Wasser gewaschen und getrocknet werden.

Das erfindungsgemäße Verfahren stellt eine leistungsfähige,
aber schonende Methode zur Herstellung von Hydrierungsprodukten der verschiedensten Art dar. Durch geeignete Wahl
der Reaktionsbedingungen sowie durch die Auswahl des
Promotors können hierbei auch selektive Hydrierungen
durchgeführt werden.

Geeignete Ausgangsmaterialien für das erfindungsgemäße
Verfahren stellen Verbindungen mit C-C-Mehrfachbindungen,
insbesondere Doppelbindungen, dar, die in offenkettigen oder cyclischen Molekülen vorliegen können, beispielsweise
auch in aromatischen Systemen, wie in Benzol-, Pyridin-
oder Furan-Systemen. Weiterhin ist das erfindungsgemäße
Verfahren zur Hydrierung der verschiedensten Oxo-Verbindungen geeignet, wobei ebenfalls selektive Hydrierungen
möglich sind, beispielsweise lediglich die Hydrierung
einer Ketogruppe zur entsprechenden Alkoholgruppe.
Aber auch die Hydrierung von Stickstoffunktionen kann
vorteilhaft nach dem erfindungsgemäßen Verfahren erfolgen,
so beispielsweise die Überführung einer Nitril-, Nitro-
oder Oxim-Gruppe in das entsprechende primäre Amin, aber
auch Hydrogenolysen von beispielsweise Azoverbindungen zu
den zugrundeliegenden Aminen.

Die Erfindung wird an den folgenden Beispielen näher
erläutert. Prozentangaben beziehen sich auf die Masse
des Ausgangsmaterials, sofern nichts anderes angegeben
ist.

Beispiel 1

4 Mol Propargylalkohol werden unter Zusatz von 1 % Nickelkatalysator bei 40 bar hydriert. Die Reaktion springt bei
80°C an und wird bei 85 - 90°C weitergeführt. Nach ca.
50 Minuten Reaktionszeit wird Propanol in einer Ausbeute
von über 97 % der Theorie erhalten.

Wird dem Ansatz erfindungsgemäß 1 % Trilaurylphosphin
zugesetzt, so springt die Reaktion bereits bei 70°C an
und ist bei einer Reaktionstemperatur von 85 bis 90°C
bereits nach 20 Minuten beendet. Das Propanol wird ebenfalls in einer Ausbeute von über 97 % der Theorie erhalten.

Beispiel 2

Analog Beispiel 1 werden 4 Mol Maleinsäurediäthylester,
jedoch bei 25 bar, hydriert. Die Reaktion springt bei
86°C an und wird bei 95 - 100°C weitergeführt. Nach

- 6 -

einer Reaktionszeit von 35 Minuten wird Bernsteinsäurediäthylester in einer Ausbeute von über 96 % der Theorie erhalten.

Werden dem Reduktionsansatz erfindungsgemäß 2 % Trilaurylphosphin zugesetzt, so springt die Reaktion bereits bei 70°C an und ist bei einer Reaktionstemperatur von 95 - 100°C bereits nach 15 Minuten beendet. Die Ausbeute beträgt ebenfalls über 96 % der Theorie.

## Beispiel 3

Analog Beispiel 1 werden 8 Mol Cyclohexen bei einem Druck von 40 bar hydriert. Die Reaktion springt bei 100°C an und wird bei 120 - 123°C weitergeführt. Nach einer Reaktionszeit von etwa 120 Minuten wird Cyclohexan in einer Ausbeute von über 97 % erhalten.

Wird dem Reaktionsansatz erfindungsgemäß 1 % Tributylphosphinoxid zugesetzt, so springt die Reaktion bereits bei 85°C an und ist bei einer Reaktionstemperatur von 120 - 123°C bereits nach 55 Minuten beendet. Es werden ebenfalls über 97 % der Theorie an Cyclohexan erhalten.

## Beispiel 4

2 Mol Benzol werden unter Zusatz von 500 g Methanol und der nachstehend genannten Menge an Katalysator bei 45 bar und einer Reaktionstemperatur von 118 - 120°C hydriert. In jedem Fall beträgt die Ausbeute an Cyclohexan über 95 % der Theorie. Die Ergebnisse zeigt die folgende Tabelle:

0008727

## Tabelle 1

| | Katalysator | % TLP* | Anspringtemp. (°C) | Reaktionszeit (min) |
|---|---|---|---|---|
| | 1 % Raney-Ni | 0 | 114 | 120 |
| a) | 1 % Raney-Ni | 1 | 85 | 50 |
| | 1 % Rh | 0 | 100 | 70 |
| b) | 1 % Rh | 1 | 88 | 40 |
| | 1 % Pd | 0 | 105 | 90 |
| c) | 1 % Pd | 1 | 88 | 40 |
| | 0,25 % Pt | 0 | 114 | 100 |
| d) | 0,25 % Pt | 1 | 102 | 45 |
| | 0,25 % Ru | 0 | 116 | 110 |
| e) | 0,25 % Ru | 1 | 105 | 50 |

\* TLP = Trilaurylphosphin

### Beispiel 5

2 Mol Phenol werden unter Zusatz von 600 g Methanol und 1 % Nickel bei 50 bar hydriert. Die Reaktion springt bei 138°C an und wird bei 140 - 142°C weitergeführt. Nach einer Reaktionszeit von 205 Minuten wird Cyclohexanol in einer Ausbeute von über 95 % der Theorie erhalten.

Wird dem Ansatz erfindungsgemäß 1 % Trilaurylphosphin zugesetzt, so springt die Reaktion schon bei 121°C an und ist bei einer Reaktionstemperatur von 140 - 142°C bereits nach 95 Minuten beendet. Das Cyclohexanol wird ebenfalls in einer Ausbeute von über 95 % der Theorie erhalten.

### Beispiel 6

2 Mol Pyridin werden unter Zusatz von 500 g Methanol und 1 % Nickelkatalysator unter einem Druck von 55 bar hydriert. Die Reaktion springt bei 132°C an und wird bei

146 - 148°C weitergeführt. Nach einer Reaktionszeit von 165 Minuten wird Piperidin in einer Ausbeute von über 94 % der Theorie erhalten.

Wird erfindungsgemäß dem Ansatz 1 % Trioctylphosphin zugesetzt, so springt die Reduktion bereits bei 104°C an und ist bei einer Reaktionstemperatur von 146 - 148°C schon nach 90 Minuten beendet. Die Ausbeute an Piperidin beträgt über 95 % der Theorie.

Beispiel 7

10 Mol Furan werden unter Zusatz von 1 % Nickelkatalysator bei einem Druck von 40 bar hydriert. Die Reaktion springt bei 68°C an und ist bei einer Reaktionstemperatur von 95 - 100°C nach 120 Minuten beendet. Tetrahydrofuran wird in einer Ausbeute von über 97 % der Theorie erhalten.

Wird dem Reduktionsansatz erfindungsgemäß 1 % Trioctylphosphin zugesetzt, so springt die Reaktion bereits bei 68°C an und ist bei einer Temperatur von 95 - 100°C bereits nach 60 Minuten beendet. Das Tetrahydrofuran wird ebenfalls in einer Ausbeute von über 97 % der Theorie erhalten.

Beispiel 8

10 Mol Propionaldehyd werden unter Zusatz von 1 % Nickel bei einem Druck von 40 bar hydriert. Die Reaktion springt bei 88°C an und ist bei einer Temperatur von 92 - 94°C nach 55 Minuten beendet. Propanol wird in einer Ausbeute von über 97 % der Theorie erhalten.

Wird dem Ansatz erfindungsgemäß 1 % Trioctylphosphin zugesetzt, so springt die Reduktion bereits bei 75°C an und ist bei 92 - 94°C bereits nach 25 Minuten beendet. Das Propanol wird ebenfalls in einer Ausbeute von über 97 % der Theorie erhalten.

## Beispiel 9

8 Mol Benzaldehyd wird unter Zusatz von 1 % Nickel bei einem Druck von 40 bar hydriert. Die Reaktion springt bei 85°C an und ist bei einer Temperatur von 96 - 100°C nach 65 Minuten beendet. Benzylalkohol wird in einer Ausbeute von über 96 % der Theorie erhalten.

Wird dem Ansatz erfindungsgemäß 1 % Tributylphosphinoxid zugesetzt, so springt die Reduktion bereits bei 71°C an und ist bei 96 - 100°C schon nach 30 Minuten beendet. Der Benzylalkohol wird ebenfalls in einer Ausbeute von 96 % der Theorie erhalten.

## Beispiel 10

10 Mol Methyläthylketon wird unter Zusatz von 1 % Nickelkatalysator bei einem Druck von 45 bar hydriert. Die Reduktion springt bei 90°C an und ist bei 95 - 100°C nach 70 Minuten beendet. Butanol-(2) wird in einer Ausbeute von über 97 % der Theorie erhalten.

Wird erfindungsgemäß unter Zusatz von 1 % Trilaurylphosphin gearbeitet, so springt die Reduktion bei 71°C an und ist bei 95 - 100°C schon nach 30 Minuten beendet. Die Ausbeute an Butanol-(2) beträgt ebenfalls über 97 % der Theorie.

## Beispiel 11

1 Mol Benzophenon wird unter Zusatz von 700 g Methanol und 1 % Nickelkatalysator bei einem Druck von 45 bar hydriert. Die Reduktion springt bei 67°C an und ist bei 98 - 100°C nach 40 Minuten beendet. Man erhält in selektiver Reaktion Diphenylcarbinol in einer Ausbeute von über 95 % der Theorie.

Wird erfindungsgemäß dem Ansatz 1 % Trilaurylphosphin zugesetzt, springt die Reduktion bei 68°C an und ist bei 98 - 100°C schon nach 20 Minuten beendet. Es wird ebenfalls Diphenylcarbinol in einer Ausbeute von über 95 % der Theorie erhalten.

Beispiel 12

10 Mol Cyclohexanon werden unter Zusatz von 1 % Nickelkatalysator bei einem Druck von 40 bar hydriert. Die Reduktion springt bei 98°C an und ist bei 110 - 112 °C nach 60 Minuten beendet. Cyclohexanol wird in einer Ausbeute von über 96 % der Theorie erhalten.

Wird dem Ansatz erfindungsgemäß 1 % Trioctylphosphin zugesetzt, so springt die Reduktion bei 74°C an und ist bei 110 - 112°C nach 25 Minuten beendet. Es wird ebenfalls Cyclohexanol in einer Ausbeute von über 96 % der Theorie erhalten.

Beispiel 13

1 Mol Chinon wird unter Zusatz von 500 g Methanol mit 1 % Nickelkatalysator bei einem Druck von 45 bar hydriert. Die Reduktion springt bei 98°C an und ist bei 110 - 115°C nach 60 Minuten beendet. In der selektiven Reduktion wird Hydrochinon in einer Ausbeute von über 93 % der Theorie erhalten.

Wird dem Ansatz erfindungsgemäß 1 % Trilaurylphosphin zugesetzt, so springt die Reduktion bei 72°C an und ist bei 110 - 115°C nach 25 Minuten beendet. Es werden ebenfalls über 93 % der Theorie an Hydrochinon erhalten.

Beispiel 14

1 Mol Acetessigsäure-p-phenetidid wird unter Zusatz von 935 g Isopropanol und 2,5 % Nickelkatalysator bei einem

Druck von 135 bar hydriert. Die Reduktion springt bei 128°C an und ist bei 135°C nach 60 Minuten beendet. 3-Hydroxybuttersäure-p-phenetidid wird in einer Ausbeute von 95 % der Theorie erhalten.

Wird die Hydrierung bei 45 bar durchgeführt, so springt die Reduktion ebenfalls bei 128°C an, dauert aber bei 135°C 120 Minuten. Die Ausbeute ist ebenfalls 95 % der Theorie.

Werden dem Reduktionsansatz erfindungsgemäß 2,5 % Trioctylphosphinoxid zugesetzt, so springt bei einem Druck von 45 bar die Reduktion bereits bei 75°C an und ist bei einer Temperatur von 135°C schon nach 20 Minuten beendet. Das 3-Hydroxybuttersäure-p-phenetidid wird ebenfalls in 95 %iger Ausbeute erhalten.

Beispiel 15

10 Mol Furfural wird unter Zusatz von 1 % Nickelkatalysator bei einem Druck von 50 bar hydriert. Die Reduktion springt bei 116°C an, verläuft aber auch bei einer Temperatur von 140°C schleppend und die Wasserstoffaufnahme bricht nach einiger Zeit ab.

Wird der Versuch bei einem Druck von 100 bar wiederholt, so springt die Reduktion bei 100°C an und erfordert bei einer Temperatur von 140°C 110 Minuten. Es wird Tetrahydrofurfurylalkohol in einer Ausbeute von über 95 % der Theorie erhalten.

Wird dem Ansatz erfindungsgemäß 1 % Trioctylphosphin zugesetzt, so springt bei einem Druck von 50 bar die Reduktion bereits bei 90°C an und ist bei einer Temperatur von 140°C nach 100 Minuten beendet. Man erhält in ebenfalls über 95 %iger Ausbeute Tetrahydrofurfurylalkohol.

Beispiel 16

2 Mol Benzonitril werden unter Zusatz von 700 g Xylol und 2,5 % des nachstehend genannten Katalysators sowie gegebenenfalls 2,5 % des genannnten Promotors bei einem Druck von 45 bar hydriert. Die Ausbeuten an Benzylamin zeigt die nachstehende Tabelle.

Tabelle 2

| Katalysator | | Promotor* | An-spring-temp. ($^{o}$C) | Reak-tions-temp. ($^{o}$C) | Reaktions-zeit (min) | Ausbeute (% d.Th.) |
|---|---|---|---|---|---|---|
| | Ni | - | 85 | 105 | 85 | >94 |
| a) | Ni | TPP | 70 | 105 | 15 | >94 |
| b) | Ni | TBPO | 78 | 105 | 20 | >94 |
| c) | Ni | TOPO | 83 | 105 | 20 | >94 |
| | Co | - | - | 140 | - | - |
| d) | Co | TPP | 128 | 140 | 60 | >94 |
| e) | Co | TBPO | 130 | 140 | 60 | >94 |
| | Pd | - | 80 | 105 | 55 | >94 |
| f) | Pd | TPP | 70 | 105 | 30 | >94 |
| | Pd gealtert | - | - | 105 | - | - |
| g) | Pd gealtert | TPP | 65 | 105 | 60 | >94 |
| | Ru | - | 80 | 105 | 70 | >94 |
| h) | Ru | TPP | 75 | 105 | 35 | >94 |
| i) | Ru | TOPO | 78 | 105 | 40 | >94 |

* TPP = Triphenylphosphin
TBPO = Tributylphosphinoxid
TOPO = Trioctylphosphinoxid

Beispiel 17

2 Mol Phenylacetonitril werden unter Zusatz von 800 g Xylol mit 2 % Nickelkatalysator bei einem Druck von 45 bar hydriert. Die Reduktion springt bei 85°C an und ist bei einer Temperatur von 105°C nach 80 Minuten beendet. 2-Phenyläthylamin wird in einer Ausbeute von über 94 % der Theorie erhalten.

Werden dem Ansatz erfindungsgemäß 2 % Tributylphosphinoxid zugesetzt, so springt die Reduktion bei 70°C an und ist bei 105°C schon nach 20 Minuten beendet. Das Phenäthylamin wird ebenfalls in Ausbeute von über 94 % der Theorie erhalten.

Beispiel 18

1 Mol Azobenzol wird unter Zusatz von 700 g Methanol und 1 % Nickelkatalysator bei einem Druck von 45 bar hydriert. Die Reduktion springt bei 88°C an und ist bei 98 - 100°C nach 40 Minuten beendet. Anilin wird in einer Ausbeute von über 94 % der Theorie erhalten.

Wird dem Ansatz erfindungsgemäß 1 % Trioctylphosphin zugesetzt, so springt die Reduktion bei 65°C an und ist bei 98 - 100°C nach 20 Minuten beendet. Das Anilin wird ebenfalls in einer Ausbeute von über 94 % der Theorie erhalten.

Beispiel 19

7 Mol 2-Nitropropan werden unter Zusatz von 1 % Nickelkatalysator bei 40 bar hydriert. Die Reduktion springt bei 87°C an und ist bei 100 - 110°C nach 80 Minuten beendet. 2-Aminopropan wird in einer Ausbeute von über 97 % der Theorie erhalten.

Wird dem Ansatz erfindungsgemäß 1 % Trioctylphosphin zugefügt, so springt die Reduktion bei 55°C an und ist bei 100 - 110°C nach 30 Minuten beendet. Die Ausbeute an 2-Aminopropan beträgt ebenfalls über 97 % der Theorie.

## Beispiel 20

1 Mol 3-Brom-nitrobenzol wird unter Zusatz von 500 g Methanol und 1 % des nachstehend genannten Katalysators sowie gegebenenfalls 2 % des genannten Promotors bei einem Druck von 45 bar hydriert. Die Ausbeuten an 3-Bromanilin zeigt die nachfolgende Tabelle:

### Tabelle 3

| | Kataly-sator | Promotor* | An-spring-temp. ($^o$C) | Reak-tions-temp. ($^o$C) | Reak-tions-zeit (min) | Aus-beute (%d.Th) | Bromab-spaltung (%) |
|---|---|---|---|---|---|---|---|
| | Pd | - | 85 | 98-100 | 35 | 91 | 4,5 |
| a) | Pd | TOP | 70 | 98-100 | 15 | 93 | 2,5 |
| b) | Pd | TOP | 70 | 75 | 40 | >95 | <0,3 |
| c) | Ni | TOP | 75 | 75 | 50 | >95 | <0,3 |

*TOP = Trioctylphosphin

## Beispiel 21

2 Mol Cyclohexanonoxim werden unter Zusatz von 500 g Methanol und 1 % Nickelkatalysator bei 40 bar hydriert. Die Reduktion springt bei 85°C an und ist bei 112 - 114°C nach 45 Minuten beendet. Die Ausbeute an Cyclohexylamin beträgt über 96 % der Theorie.

Wird dem Ansatz erfindungsgemäß 1 % Trioctylphosphin zugesetzt, so springt die Reduktion bei 75°C an und ist bei 112 - 114°C nach 20 Minuten beendet. Das Cyclohexylamin wird ebenfalls in einer Ausbeute von über 96 % der Theorie erhalten.

Patentansprüche:

1. Verfahren zur katalytischen Hydrierung von organischen Verbindungen in flüssiger Phase unter Zusatz einer organischen Phosphorverbindung als Promotor, dadurch gekennzeichnet, daß als Promotor ein organisches Phosphin oder Phosphinoxid eingesetzt wird, wobei die Hydrierung von N-Acetoacetylarylamiden in Gegenwart von Phosphinen ausgenommen ist.

2. Verfahren nach Anspruch 1, dadurch gekennzeichnet, daß als Promotor ein Phosphin oder Phosphinoxid eingesetzt wird, das drei gleiche Alkylreste mit 4 bis 12 Kohlenstoffatomen trägt.

3. Verfahren nach Anspruch 1, dadurch gekennzeichnet, daß Triphenylphosphin als Promotor eingesetzt wird.

## Europäisches Patentamt

## EUROPÄISCHER RECHERCHENBERICHT

| Kategorie | EINSCHLÄGIGE DOKUMENTE Kennzeichnung des Dokuments mit Angabe, soweit erforderlich, der maßgeblichen Teile | betrifft Anspruch |
|---|---|---|
| X | FR - A - 1 514 495 (MONTECATINI)<br>  * Seite 2, Beispiel 1; Ansprüche 1-3 *<br>-- | 1,3 |
|  | US - A - 3 480 659 (DEWHIRST-SHELL)<br>  * Spalte 1, Zusammenfassung *<br>-- | 1,3 |
|  | US - A - 3 454 644 (DEWHIRST-SHELL)<br>  * Spalte 1, Zusammenfassung *<br>-- | 1,3 |
|  | US - A - 3 366 646 (DEWHIRST-SHELL)<br>  * Spalte 1, Zusammenfassung *<br>-- | 1,3 |
|  | US - A - 3 489 786 (DEWHIRST-SHELL)<br>  * Ansprüche 1-4; Spalten 6-8; Beispiele 2-4 und 8 *<br>-- | 1,3 |
|  | FR - A - 2 343 720 (HOECHST)<br>  * Anspruch 1 * | 1-3 |
| D | & DE - A - 2 609 835<br>-- |  |
| A | GB - A - 1 011 835 (ESSO)<br>  * Ansprüche 13,14; Seite 2, Zeilen 1-32 *<br>-- | 1 |

### KLASSIFIKATION DER ANMELDUNG (Int Cl³)

C 07 B  1/00
B 01 J 31/02//
C 07 C  5/03
        5/10
       13/18
       29/14
       29/17
       29/20
       29/136
C 07 C 31/10
       31/12
       33/10
       33/12
       37/07

### RECHERCHIERTE SACHGEBIETE (Int Cl³)

C 07 B  1/00
B 01 J 31/02

### KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 28-11-1979 | DE ROY |

EPA form 1503.1  06.78

**Europäisches Patentamt**

## EUROPÄISCHER RECHERCHENBERICHT

| | EINSCHLÄGIGE DOKUMENTE | | | KLASSIFIKATION DER ANMELDUNG (Int.Cl. 3) |
|---|---|---|---|---|
| Kategorie | Kennzeichnung des Dokuments mit Angabe, soweit erforderlich, der maßgeblichen Teile | | betrifft Anspruch | |
| D,A | US - A - 3 642 658 (ALLUM et al. B.P.)  * Spalte 1, Zusammenfassung *  -- | | 1 | C 07 C   39/08 67/303 69/40 85/11 85/12 C 07 D 295/02 307/08 307/12 |
| A | US - A - 3 130 237 (WALD-SHELL)  * Ansprüche 1-3,5-9 *  -- | | 1 | |
| A | US - A - 3 219 738 (KOTHARI et al. GOODYEAR)  * Spalte 1, Zusammenfassung *  -- | | 1 | RECHERCHIERTE SACHGEBIETE (Int. Cl. 3) |
| A | US - A - 3 230 250 (SPEZIALE-MONSANTO)  * Spalte 1, Zeilen 14-43 *  -- | | 1 | |
| A | QUARTERLY REVIEW 22, 1968, Nr. 2, Seiten 222-251 J.I.G. CADOGAN: "Reduction of Nitro- and Nitroso-compounds by Tervalent Phosphorus Reagents"  * Seite 222, Absätze 3-4; Seite 247, Schema 34; Seite 248, Schemas 36,37; Vergleichung Nr. 26-28 *  ---- | | 1 | |